# EUROPÄISCHE PATENTSCHRIFT

(11) **EP 0 586 621 B1**
(45) Veröffentlichungstag und Bekanntmachung des Hinweises auf die Patenterteilung: **30.08.2000**
(21) Anmeldenummer: 92924464.8
(22) Anmeldetag: 14.05.1992
(51) Int. Cl.: A61K 31/535

(54) **LINSIDOMIN ZUR BEHANDLUNG EREKTILER DYSFUNKTIONEN**
LINSIDOMIN FOR TREATING ERECTILE DYSFUNCTIONS
LINSIDOMINE POUR LE TRAITEMENT DE DYSFONCTIONNEMENTS ERECTILES

(30) Priorität: 27.05.1991 DE 4117249
(43) Veröffentlichungstag der Anmeldung: 16.03.1994
(73) Patentinhaber: Stief, Christian, Dr., D-30966 Hemmingen-Westerfeld (DE)
(72) Erfinder: Stief, Christian, Dr., D-30966 Hemmingen-Westerfeld (DE)
(74) Vertreter: Koepe, Gerd L., Dipl.-Chem.
(86) Internationale Anmeldenummer: EP9201109
(87) Internationale Veröffentlichungsnummer: WO9221346

(56) Entgegenhaltungen:
- US-A- 3 312 690
- The Journal of Urology, (AUA Eighty-Seventh Annual Meeting, Washington, DC, 10. -14. Mai 1992), Band 147, Nr. 45, 1992, C.G. STIEF et al.: "Prelimary results with the nitric oxyde (NO) donor SN-1 in the treatment of human erectile dysfunction", Seite 265A, siehe das ganze Dokument
- World Journal of Urology, Band 9, Nr. 4, 1991, C.G. STIEF et al.: "Preliminary report on the effect of the nitric oxide donor SIN-1 on human cavernous tissue in vivo", Seiten 237-239, siehe das ganze Dokument
- F. HOLMQUIST: "Nonadrenergic, Noncholinergic Mechanisms for Contraction and Relaxation of Penile Smooth Muscle", 20. September 1991, Thesis, Lund University, siehe Seiten 109-130
- The Journal of Physiology, (Royal Free Meeting, 15. - 16. Februar 1991), Band 438, 1991, R.S. PICKARD et al.: "Evidence that nitric oxide mediates human penile erection", Seite 103P, siehe das ganze Dokument
- The FASEB Journal, Band 5, Nr. 4, 11. März 1991, P.A. BUSH et al.: "Nitric oxide is responsible for nonadrenergic noncholinergic (NANC) relaxation of corpus cavernosum leading to penile erection in rabbit and man", Seite A400, siehe das ganze Dokument
- Acta Physiologica Scandinavica, Band 141, Nr. 3, März 1991, F. HOLMQUIST et al.: "L-Ng-nitro arginine inhibits non-adrenergic, non-cholinergic relaxation of human isolated corpus cavernosum", Seiten 441-442, siehe das ganze Dokument
- Blood Vessels, Band 27, 1990, J. REDEN: "Molsidomine", Seiten 282-294, siehe das ganze Dokument
- Urologe A, Band 25, 1986, C.G. STIEF et al.: "Schwellkörper-Autoinjektionstherapie (SKAT): erste Erfahrungen bei erektiler Dysfunktion", Seiten 63-66, siehe das ganze Dokument (in der Anmeldung erwähnt)

## Beschreibung

Die Erfindung betrifft die Verwendung von Linsidomin sowie seiner pharmakologisch verträglichen Salze bei der Behandlung erektiler Dysfunktionen.

Etwa 5 % der Männer im 40. Lebensjahr und 20 % im 60. Lebensjahr leiden an einer erektilen Dysfunktion. Durch den Verlust der Potenz wird das körperliche, seelische und soziale Selbstverständnis des Mannes, insbesondere des jungen Mannes, erschüttert. Patienten mit chronischer erektiler Dysfunktion sind in Ihrer Sexualität und Persönlichkeit verunsichert und als krank anzusehen.

Zur Behandlung von bis in die siebziger Jahre überwiegend auf psychische Ursachen zurückgeführten Potenzstörungen wurden neben psychotherapeutischen Maßnahnen Testosterone oder Aphrodisiaka von umstrittenem Wert eingesetzt. Erst durch die Erforschung der Physiologie des Erektionsablaufs wurde festgestellt, daß bei mehr als 60% der Patienten organische Ursachen die Erektionsstörungen bewirken, wobei autonome Efferenzen aus dem parasympathischen Anteil des Sakralmarkes, Neurotransmitter, Dilatation der Penisarterien, Relaxation der cavernösen Räume und Konstriktion der Venen eine Rolle spielen. In über 70% der Fälle sind vaskuläre Faktoren ursächlich beteiligt, wie pathologische arterielle Blutversorgungen oder abnorm gesteigerter venöser Abfluß aus den kavernosen Räumen. Neurogene Störungen sind zu etwa 20% beteiligt.

Die orale Therapie dieser organisch bedingten Dysfunktionen mit vasoaktiven Substanzen wie Yohimbin, Phenoxybenzamin, Terbutalin, Bethanechol, Levodopa, Verapamil oder Theophyllin erwies sich als erfolglos. Neben der Anwendung von Prothesenimplantationen oder Revaskularisierungsoperationen erwies sich eine intrakavernöse Injektion von Papaverin (Virag, Lancet, 2, 938, 1982), dem α-Rezeptorenblocker Phenoxybenzamin (Brindley, Br. J. Psychiatr., 143, 332, 1983) und eine Kombination von Papaverin und dem α-Rezeptorenblocker Phentolamin (Stief, Urologe A, 25, 63, 1986) als erfolgreich. Letztere Therapiemethode läßt sich vom Patienten selbständig durchführen und wird auch als Schwellkörper-Autoinjektionstherapie (SKAT) bezeichnet.

Nachteilig erwiesen sich jedoch eine teilweise unerwünscht verlängerte Erektion mit der Gefahr des Priapismus bei der Verwendung von Papaverin, unerwünschte Schmerzhaftigkeit bei der Anwendung von Phenoxybenzamin sowie eine mögliche Cancerogenität dieser Verbindung.

Im Tierexperiment (Cynomolgus) wurden zudem bei 1-2 Intracavernös-Injektionen Papaverin wöchentlich über 12 Monate ausgedehnte Fibrosierungen weiter Teile des Schwellkörpers festgestellt, was beim Menschen zu äußerst negativen Langzeitergebnissen führen würde, da bei einer Fibroisierung des corpus cavernosum keine Erektion mehr erfolgen kann.

In dem Dokument "J. Physiol. (1991), 438, 103P" wird beschrieben, daß im in-vitro-Versuch L-NOARG NO freisetzt, das seinerseits einen Einfluß auf die Erektion hat. Linsidomin kann ebenfalls NO freisetzen; jedoch ist dem Dokument im Hinblick auf die erheblichen Struktur-Unterschiede zwischen L-NOARG und Linsidomin nichts zu einem Einfluß von Linsidomin auf die erektile Dysfunktion zu entnehmen.

Die Druckschrift US-A 3,312,690 offenbart die Verwendung von Sydnonimin-Derivaten wie beispielsweise 3-Morpholinosydnonimin (= Linsidomin) als Muskelrelaxantien und Mittel zum Erweitern der Coronargefäße und peripheren Gefäße; eine Verwendung zur Bekämpfung der erektilen Dysfunktion ist dem Dokument nicht zu entnehmen.

Die Anwendung von Acetylcholin ist bei nur kurzzeitiger Erektion mit starken systemischen Nebenwirkungen verbunden, und die Injektion von Prostaglandin E₁ wird von den Patienten wegen zu großer Schmerzhaftigkeit abgelehnt.

Aufgabe der Erfindung war daher die Entwicklung und Herstellung von Arzneimitteln zur Behandlung von neurogenen, arteriellen, neurotransmitterbedingten, myopathischen, venösen oder psychogenen erektilen Dysfunktionen bei Säugetieren, bevorzugt beim Menschen, ohne die genannten Nebenwirkungen. Überraschenderweise wurde nun gefunden, daß die intracavernöse Injektion von Linsidomin-Hydrochlorid zu einer Erektion mit vollständiger Rigidität führt.

Die Erfindung betrifft daher die Verwendung von 3-Morpholinosydnonimin (Linsidomin, SIN-1) sowie von dessen pharmakologisch verträglichen Salzen, bevorzugt von Linsidomin-Hydrochlorid, zur Herstellung von Arzneimitteln zur Behandlung erektiler Dysfunktionen bei Säugetieren, bevorzugt beim Menschen.

Die Erfindung betrifft auch die Verwendung des aus galenischen Gründen in die pharmakologisch verträglichen Salze übergeführten Linsidomins. Die Salze werden in üblicher Weise durch Neutralisation der Base mit anorganischen oder organischen Säuren erhalten.

Als anorganische Säuren kommen zum Beispiel Salzsäure, Schwefelsäure, Phosphorsäure oder Bromwasserstoffsäure, als organische Säuren zum Beispiel Carbon-, Sulfo- oder Sulfonsäuren wie Essigsäure, Weinsäure, Milchsäure, Propionsäure, Glykolsäure, Malonsäure, Maleinsäure, Fumarsäure, Gerbsäure , Bernsteinsäure, Alginsäure, Benzoesäure, 2-Phenoxybenzoesäure, 2-Acetoxybenzoesäure, Zimtsäure, Mandelsäure, Citronensäure, Äpfelsäure, Salicylsäure, 3-Aminosalicylsäure, Ascorbinsäure, Embonsäure, Nicotinsäure, Isonicotinsäure, Oxalsäure, Aminosäuren, Methansulfonsäure, Ethansulfonsäure, 2-Hydroxyethansulfonsäure, Ethan-1,2-disulfonsäure, Benzolsulfonsäure, 4-Methylbenzolsulfonsäure oder Naphtalin-2-sulfonsäure in Frage.
Die erfindungsgemäß verwendeten Arzneimittel enthalten neben den üblichen Hilfs-, Träger- und Zusatzstoffen eine wirksame Dosis von Linsidomin oder eines seiner Salze zur Behandlung der genannten Dysfunktionen. Die Dosierung ist abhängig von Spezies, Körpergewicht, Alter, individuellem Zustand und Applikationsart.

Als Applikationsformen kommen sowohl parenterale als auch topische Zubereitungen wie beispielsweise Lotionen, Cremes, Lösungen, Gele, Sprays, elastische Flüssig-Pflaster, transdermale Systeme oder Beschichtungen für Kondome in Frage.

Bevorzugt ist eine parenterale Applikation, da sie die sicherste Form ist, eine gezielte Wirkstoffmenge direkt ohne weiter Verfälschung durch andere Faktoren gezielt einzusetzen.

Die Erfindung betrifft daher auch eine Einmal-Arzneimittelpackung, enthaltend in steriler Abpackung eine Einmalspritze mit einer parenteralen Zubereitung einer Dosiseinheit eines erektionsfördernden Wirkstoffes aus der Gruppe Linsidomin und seine pharmakologisch verträglichen Salze oder aus der Gruppe Linsidomin und seine pharmakologisch verträglichen Salze in Wirkstoffkombination mit synergistisch wirkenden Substanzen aus der oben genannten Gruppe, mit Desinfektionsmitteln versehene sterile Tupfer sowie Anweisungen zur Verwendung.

Besonders bevorzugt zur vereinfachten Anwendung ist also ein steriler Applikationsatz zur einmaligen Anwendung, welcher neben sterilen, mit Desinfektionslösungen versehenen Tupfern und der zugehörigen Benutzungsinformation Einmalspritzen zur vereinfachten Anwendung mit einer Dosiseinheit einer wirksamen Menge, bevorzugt einer parenteralen Zubereitungsform, von Linsidomin oder seiner pharmakologisch verträglichen Salze, enthält. Die genannten Einmal-Fertigspritzen sind vorzugsweise vom Typ Ultrafein, wie sie beispielsweise in der Insulintherapie zur Anwendung kommen. Bevorzugt sind auch Spritzen in der Art von Autoinjektoren, die noch einfacher in ihrer Handhabung sind. Die Fertigspritzen sind in bevorzugter Form in lichtundurchlässiger ausgeführt, da sich dadurch der Zusatz von Photostabilisatoren zu den parenteralen Zubereitungen erübrigt. Sie können jedoch auch so ausgebildet sein, daß Wirkstoff und zugehöriges Injektionslösungsmittel erst direkt vor der Injektion gemischt werden. Dies kann zum Beispiel in Zwei-Behältnis-Ampullen bzw. -Spritzen geschehen. Dadurch kann sich dann ein gegebenenfalls erforderlicher Photostabilisator-Zusatz ebenfalls erübrigen.

Die sterilen Tupfer zur Desinfektion enthalten die üblichen Mittel zur Haut-Desinfektion wie beispielsweise Ethanol. Sie können jedoch auch noch zusätzlich erektionsfördernde Mittel in einer topisch applizierbaren Form zur Steigerung bzw. Verlängerung der Wirkung enthalten.

Zubereitungen zur parentalen Applikation enthalten 0,1 bis 5 mg, bevorzugt 0,1 bis 2 mg, Linsidomin pro Dosiseinheit und können in separaten Dosiseinheitsformen wie z.B. Ampullen oder Vials vorliegen. Vorzugsweise werden Lösungen des Wirkstoffes verwendet, bevorzugt wässrige Lösungen, aber auch Suspensionen. Diese Injektionsformen können als Fertigpräparat zur Verfügung gestellt werden oder erst direkt vor der Anwendung durch Mischen der wirksamen Verbindung, zum Beispiel des Lyophilisats, gegebenenfalls mit weiteren festen Trägerstoffen, mit dem gewünschten Lösungs-oder Suspensionsmittel zurbereitet werden. Für topische Applikationsformen ist selbstverständlich eine höhere Wirkstoffkonzentration als die für parenterale Zubereitungen genannte angezeigt.

Parenterale wie topische Formen können sterilisiert sein und/oder gegebenenfalls Hilfsstoffe wie Konservierungsmittel, Stabilisatoren, Netzmittel, Penetrationsmittel, Emulgatoren, Spreitmittel, Lösungsvermittler, Salze zur Regelung des osmotischen Drucks oder zur Pufferung und/oder Viskositätsregulatoren enthalten.

Derartige Zusätze sind z.B. Tartrat- und Citrat-Puffer, Ethanol, Komplexbilder (wie Ethylendiamintetraessigsäure und deren nicht-toxische Salze). Zur Regelung der Viskosität kommen hochmolekulare Polymere in Frage wie beispielweise flüssiges Polyethylenoxid, Carboxymethylcellulosen, Polyvinylpryrrodilone, Dextrane oder Gelatine. Feste Trägerstoffe sind zum Beispiel Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyethylenglykol.

Ölige Suspensionen für parenterale oder topische Anwendungen können vegetabile synthetische Öle wie beispielsweise flüssige Fettsäureester mit jeweils 8 bis 22 C-Atomen in den Fettsäureketten, zum Beispiel Palmitin-, Laurin-, Tridecyl-, Margarin-, Stearin-, Arachin-, Myristin-, Behen-Pentadecyl-, Linol-, Elaidin-, Brassidin-, Eruca- oder Ölsäure, die mit ein-bis dreiwertigen Alkoholen mit 1 bis 6 C-Atomen wie beispielsweise Methanol, Ethanol, Propanol, Butanol, Pentanol oder deren Isomeren, Glycol oder Glycerol verestert sein. Derartige Fettsäureester sind beispielsweise handelsübliche Miglyole, Isopropylmyristat, Isopropylpalmitat, Isopropylstearat, PEG-6-Caprinsäure, Capryl/Caprinsäureester von gesättigten Fettalkoholen, Polyoxyethylenglyceroltrioleate, Ethyloleat, wachsartige Fettsäureester wie künstliches Entenbürzeldrüsenfett, Kokosfettsäure-isopropylester, Ölsäureoleylester, Ölsäureredecylester, Milchsäureethylester, Dibutylphthalat, Adipinsäurediisopropylester, Polyol-Fettsäureester u.a., Ebenso geeignet sind Siliconöle verschiedener Viskosität oder Fettalkohole wie Isotridecylalkohol, 2-Octyldodecanol, Cetylstearylalkohol oder Oleylalkohol, Fettsäuren wie bespielsweise Ölsäure. Weiterhin können vegetabile Öle wie Rizinusöl, Mandelöl, Olivenöl, Sesamöl, Baumwollsaatöl, Erdnußöl oder Sojabohnenöl Verwendung finden. Die genannten Stoffe haben zudem die Eigenschaften eines Spreitmittels, das heißt, es erfolgt eine besonders gute Verteilung auf der Haut.

Als Lösungsmittel, Gelbildner und Lösungsvermittler kommen in Frage Wasser oder mit Wasser mischbare Lösungsmittel. Geeignet sind zum Beispiel Alkohole wie beispielsweise Ethanol oder Isopropylalkohol, Benzylalkohol, 2-Octyldodecanol, Polyethylenglykole, Phthalate, Adipate, Propylenglykol, Glycerin, Di- oder Tripropylenglykol, Wachse, Methylcellosolve, Cellosolve, Ester, Morpholine, Dioxan, Dimethylsulfoxid, Dimethylformamid, Tetrahydrofuran, Cyclohexanon USW..

Als Filmbildner können Celluloseether verwendet werden, die sich sowohl in Wasser als auch in organischen Lösungsmitteln lösen bzw. anquellen können und nach dem Trocknen eine Art Film bilden, wie beispielsweise Hydroxypropylcellulose, Methylcellulose, Ethylcellulose oder lösliche Stärken.

Mischformen zwischen Gel- und Filmbildnern sind durchaus ebenfalls möglich. Hier kommen vor allem ionische Makromoleküle zur Anwendung, wie beispielsweise Natriumcarboxymethylcellulose, Polyacrylsäure, Polymethacrylsäure und deren Salze, Natriumamylopektinsemiglykolat, Alginsäure oder Propylenglykol-Alginat als Natriumsalz, Gummi arabicum, Xanthan-Gummi, Guar-Gummi oder Carrageenan.

Als weitere Formulierungshilfsmittel können eingesetzt werden:
Glycerin, Paraffin unterschiedlicher Viskosität, Triethanolamin, Collagen, Allantoin, Novantisolsäure, Parfümöle.

Auch die Verwendung von Tensiden, Emulgatoren oder Netzmitteln kann zur Formulierung notwendig sein, wie zum Beispiel von Na-Laurylsulfat, Fettalkoholethersulfaten, Di-Na-N-lauryl-β-iminodipropionat, polyoxyethyliertes Rizinusöl oder Sorbitan-Monooleat, Sorbitan-Monostearat, Cetylalkohol, Lecithin, Glycerinmonostearat, Polyoxyethylenstearat, Alkylphenolpolyglykolether, Cetyltrimethylammoniumchlorid oder Mono-/ Dialkylpolyglykolether-orthophosphorsäure-mono-ethanolaminsalze.

Stabilisatoren wie Montmorillonite oder kolloidale Kieselsäuren zur Stabilisierung von Emulsionen oder zur Verhinderung des Abbaus der aktiven Substanzen wie Antioxidantien, beispielsweise Tocopherole oder Butylhydroxyanisol, oder Konservierungsmittel, wie p-Hydroxybenzoesäureester, können ebenfalls zur Zubereitung der gewünschten Formulierungen gegebenenfalls erforderlich sein.

Zur Förderung der Penetration enthalten transdermale Formulierungen vorzugsweise organische, gut hautverträgliche Lösungsmittel wie Ethanol, Methylpyrrolidon, Polyethylenglykol, Oleylalkohol, Octanol, Linolsäure, Triacetin, Propylenglykol, Glycerin, Solketal oder Dimethylsulfoxid.

Infolge der Lichtempfindlichkeit von Linsidomin ist es angezeigt, den Darreichungsformen Photostabilisatoren zuzusetzen oder sie in entsprechend lichtschützenden Verpackungen bereitzustellen, wie beispielsweise in lichtundurchlässigen Einmal-Fertigspritzen.

Als Photostabilisatoren kommen Verbindungen in Frage wie Chinoline, z.B. 8-Hydroxychinolin oder Chinolingelb; geeignete Lebensmittelfarbstoffe; Flavanoide wie z.B. Hesperidin, Hesperidin-methylchalcon, Hesperidinphosphat, Hesperitin, Quercetin, Quercitrin, Rutin, Rutinsulfat, Naringenin, Kämpferol-7,4'-dimethylether, Morin, Apigetrin, Luteolin und sein 7-Glycosid, oder Troxerutin. Sie werden dem Linsidomin im Mengenverhältnis 0,001 : 1 bis 50 : 1, vorzugsweise 0,1 : 1 bis 20 : 1, zugesetzt.

Die Herstellung, Abfüllung und die Verschließung der Präparate erfolgt unter den üblichen antimikrobiellen und aseptischen Bedingungen. Auch für topischen beziehungsweise transdermalen Einsatz erfolgt eine Abpackung möglichst in separaten Dosiseinheiten zur Erleichterung der Handhabung, auch hier wie bei parenteralen Formen gegebenenfalls aus Stabilitätsgründen durch separate Abpackung der Wirkstoffe beziehungsweise deren Kombinationen als Lyophilisat, gegebenenfalls mit festen Trägerstoffen, und den erforderlichen Lösungsmitteln etc.,

Ein weiterer Aspekt der Erfindung ist die Verwendung von Linsidomin in Kombination mit synergistisch wirkenden Substanzen wie Adenosin, Vitaminen, zum Beispiel Vitamin A oder H, Prostaglandinen, zum Beispiel E₁, mit Peptiden wie z.B. Calcitonine gene related peptides (CGRP), mit Calcium-Antagonisten wie Nifedipin, Verapamil, Diltiazem, Gallopamil, Niludipin, Nimodipin, Nicardipin, Prenylamin, Fendilin, Terodilin, Nisaldipin, Nitrendipin oder Perhexilin. Weitere Kombinationsmöglichkeiten bestehen mit α-Rezeptorenblockern, beispielsweise Phentolaminmethansulfonat, Phenoxybenzamin oder Minoxidil, oder Relaxantien der glatten Muskulatur wie Papaverin, Trinitroglycerin, Natriumnitroprussid oder S-Nitroso-N-acetylpenicillamin.

Die nachfolgenden Ausführungsbeispiele zu möglichen Arzneizubereitungen sind ausschließlich zur Veranschaulichung der erfinderischen Lehre, jedoch keineswegs als deren Limitierung gedacht.

### Beispiel 1 - Injektionslösung

400 mg Linsidomin-Hydrochlorid werden mit 750 mg NaCl in destilliertem Wasser gelöst, mit 1N HCl auf pH 3,7 eingestellt und mit destilliertem Wasser auf 100 ml aufgefüllt und in 0,5 ml-Ampullen abgepackt.

### Beispiel 2 - Lösung zur topischen Applikation

Aus 3,2 g Linsidomin-Hydrochlorid, 2 ml Isopropylmyristat und 10 ml Ethanol wird eine Lösung zur topischen Applikation bereitet und zu Dosiseinheiten von jeweils 2 ml abgepackt.

### Beispiel 3 - Transdermales Pflaster

10 g Linolsäure und 90 g Propylenglykol werden gemischt. In dieser Mischung werden 40 g Linsidomin-Hydrochlorid gelöst. Mit der Lösung werden einseitig mit Kunststoff beschichtete Gazequadrate getränkt und in Aluminiumfolie versiegelt.

### Beispiel 4 - Streichfähiges Gel

94 g gereinigtes Wasser werden auf 70°C erwärmt und mit 80 g Linsidomin-Hydrochlorid versetzt. Nach Zugabe von 0,2 g p-Hydroxybenzoesäureester werden in die erhaltene Lösung 5 g Methylhydroxyethylcellulose dispergiert. Dann wird unter Rühren abgekühlt. Nach dem Erkalten erhält man ein hochviskoses Gel mit einer Viskosität von 90 Pa.s.

### Beispiel 5 - Öl-in-Wasser-Emulsion

In einem ersten Ansatz werden 7 g einer Mischung bestehend aus gesättigten Fettsäuren, Fettalkoholen, Wollwachs, Mineralölen und nichtionogenen Emulgatoren zusammen mit 2,5 g Polyethylenglykol-Glycerolfettsäureester, 3 g Monoglyceriden der Stearin- und Palmitinsäure, 0,3 g Cetylalkohol und 3,0 g Isopropylpalmitat durch Erwärmen auf 70°C im Wasserbad homogen geschmolzen. In einem zweiten Ansatz werden 80 g gereinigtes Wasser unter Rühren mit 3 g Propylenglykol gemischt und auf 70°C erwärmt. Das so erhaltene Gemisch wird dann mit 40 g Linsidomin-Hydrochlorid und 200 mg eines Konservierungsmittels versetzt. Die erhaltene klare Lösung wird unter Rühren bei 70°C in den ersten Ansatz einemulgiert. Die so erhaltene Emulsion wird auf 40°C abgekühlt und der durch Verdunstung erlittene Wasserverlust ergänzt. Die auf 30°C abgekühlte Emulsion wird dann abgefüllt.

### Beispiel 6 - Flüssig-Pflaster

40 g Linsidomin-Hydrochlorid werden in einer Mischung von 5 g Benzylalkohol, 6 g Isopropylstearat oder einer gleiche Menge eines Isopropylmyristat/Isopropylpalmitat/-Isopropylstearat-Gemisches, 10 g Vinylpyrrolidon/-Vinylacetat-Copolymer und 89 g Isopropanol gelöst. Die Lösung kann in separaten Dosiseinheiten zur flüssigen Applikation oder als Spray mit den üblichen Treibmitteln abgepackt werden.

### Beispiel 7 - Öl-in-Wasser-Emulsion

Nach den üblichen Methoden wird eine Mischung von 40 g Linsidomin-Hydrochlorid, 9 g eines Gemisches von Mono-und Diglyceriden der Palmitin- und Stearinsäure, 3 g Cetylstearylalkohol mit ca. 12 Mol Ethylenoxid, 10 g 2-Octyldodecanol, 5 g dickflüssigem Paraffin, 5 g Benzylalkohol, 500 mg PHB-Ester und entmineralisiertem Wasser ad 100 g hergestellt.

### Beispiel 8 - Creme weicher Konsistenz

Eine derartige Creme enthält beispielsweise
40 g Linsidomin-Hdrochlorid, 4 g Mono- und Diglyceride von Palmitin und Stearinsäure, 4 g Cetylpalmitat, 1 g Cetylstearylalkohol mit ca. 12 Mol Ethylenoxid, 1 g Cetylstearylalkohol mit ca. 30 Mol Ethylenoxid, 5 g Isopropylmyristat-/Isopropylpalmitat-/Isopropylstearat-Gemisch, 0,5 g schwach vernetzte Polyacrylsäure von extrem hohem MG, 0,11 g Natriuznhydroxid 45%ig, 3 g Glycerin und entmineralisiertes Wasser ad 100 g.

### Beispiel 9 - Nichtfettende Emulsion

Eine Mischung von 2,5 g Ölsäuredecylester, 2,5 g Isopropylmyristat, 4 g dünnflüssigem Paraffin, 0,9 g Polyethylenstearat, 0,6 g Sorbitan- und Glycerinfettsäureester werden 10 min bei 70°C gerührt und geschmolzen. Die geschmolzene Mischung wird in eine 75°C warme Lösung von 50 g entmineralisiertem Wasser, 4 g Linsidomin-Hydrochlorid und 100 mg Allantoin unter Rühren gegeben und auf 45°C abgekühlt. Bei dieser Temperatur wird ein Carbopolschleim aus 10 g Ethanol, 0,7 g Carbopol® 934 (schwach vernetzte Polyacrylsäure) und 22,95 g entmineralisiertem Wasser gegeben, welche mit Turrax dispergiert wurde, anschließend 2 h gequollen ist und mit 0,15 g 45%iger Natronlauge neutralisiert wurde. Beim Erreichen von 40°C wird dann noch 1 g Collagen zugegeben. Zuletzt wird die Rohemulsion, gegebenenfalls nach einer Zugäbe von 0,6 g Parfümöl, bei 20 bis 25°C im Hochdruckhomogenisator homogenisiert.

### Beispiel 10 - Gelatine-Lösung

Für eine Gelatine-Lösung werden 2 mg Linsidomin-Hydrochlorid, 150 mg Gelatine, 4,7 mg Phenol mit destilliertem Wasser zu 1 ml aufgefüllt und in Vials zu 1 ml abgefüllt.

### Beispiel 11 - Spray

In einer Mischung von 3,5 ml Miglyol 812 und 0,08 g Benzylalkohol werden 1 g Linsidomin-Hydrochlorid suspendiert. Diese Suspension wird in einen Behälter mit Dosierventil eingefüllt. Es werden nun 5 ml Freon® 12 unter Druck durch das Ventil in den Behälter gefüllt. Durch Schütteln wird das Freon® in der Miglyol-Benzylalkoholmischung gelöst.

Die Wirksamkeit der Arzneimittel für die erfindungsgemäße Lehre wird durch folgende Studie belegt:

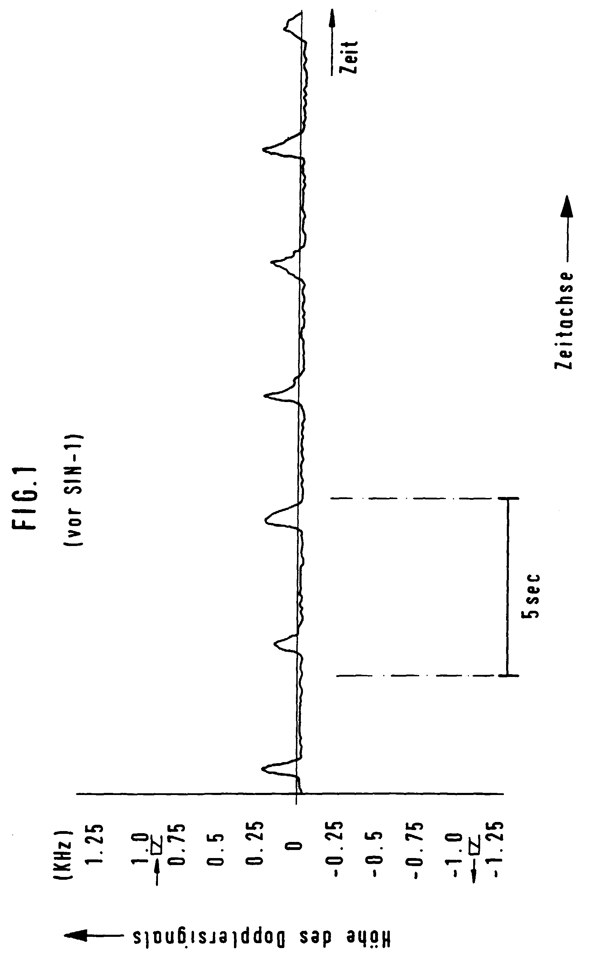

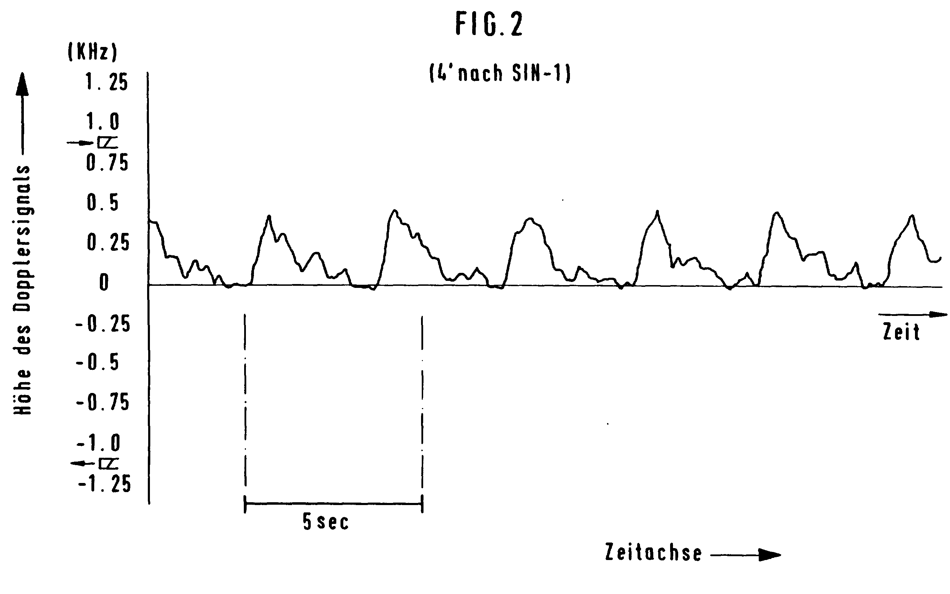

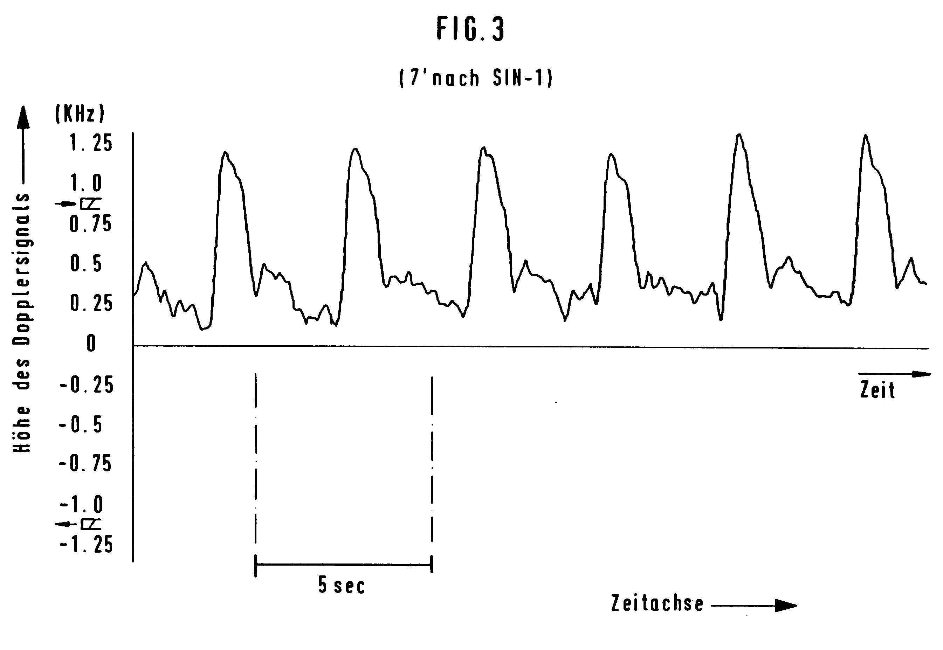

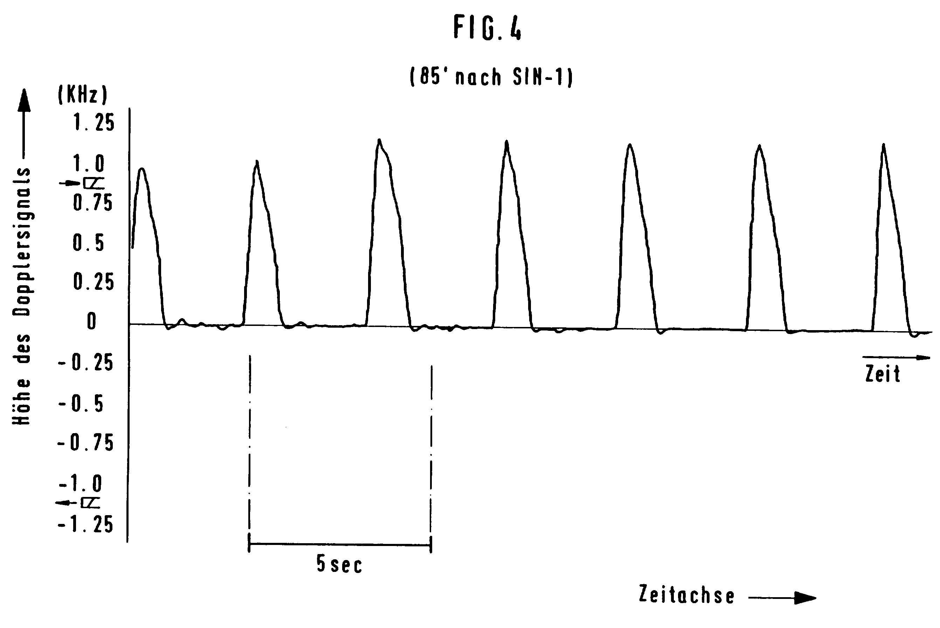

An acht Männern wurde durch die intracavernöse Injektion von 0,01 bis 2 mg Linsidomin-Hydrochlorid eine dosisabhängige Erektion ohne systemische Nebenwirkungen erzielt. Die dramatische Steigerung des arteriellen Einstroms in den Schwellkörper nach intracavernöser Injektion von 0,2 mg Linsidomin-Hydrochlorid bei einem Patienten mit erektiler Dysfunktion ist in den Abbildungen 1 bis 4 dargestellt. Die Doppler-Sonographien zeigen die Flußgeschwindigkeit des Blutes in einer Penisarterie.
Abb. 1 zeigt die normale Flußgeschwindigkeit vor Applikation von Linsidomin-Hydrochlorid.
Abb. 2 zeigt den Zustand 4 Minuten nach intracavernöser Injektion von 0,2 mg Linsidomin-Hydrochlorid in Form eines kräftigen Signals bei deutlich erhöhtem Blutfluß und beginnender Erektion.
Abb. 3 zeigt 7 Minuten nach Injektion ein sehr kräftiges Doppler-Signal bei zunehmender Erektion.
Abb. 4 zeigt 85 Minuten nach der Injektion einen immer noch deutlich erhöhten Blutfluß bei abflauender vollständiger Erektion.

Die Verwendung von Linsidomin in der Behandlung der erektilen Dysfunktion stellt auf Grund dieser Ergebnisse einen wesentlichen Fortschritt in der Schwellkörper-Autoinjektionstherapie (SKAT), der Standardbehandlungsform der erektilen Dysfunktion, dar (Stief, Urologe A, 25, 63, 1986). Die Freisetzung von Stickoxyd aus Linsidomin und damit EDRF findet auf nicht-enzymatischem Wege innerhalb des cavernösen Gewebes statt. Diese Umwandlung begrenzt die Dauer der Wirksamkeit in der Induktion einer Erektion. Damit wird die bislang gefährlichste Nebenwirkung der Schwellkörper-Autoinjektionstherapie, die prolongierte Erektion mit Gefahr der endgültigen Impotenz, ausgeschaltet. Somit bietet die Behandlung der erektilen Dysfunktion mit Linsidomin ein Optimum an therapeutischer Effektivität bei gleichzeitiger Nebenwirkungsarmut, da Linsidomin auch der aktive Metabolit des bereits seit langem in der Herz-Kreislauftherapie eingesetzten und erprobten Molsidomins ist.

## Patentansprüche

1. Verwendung von Linsidomin oder seinen pharmakologisch verträglichen Salzen zur Herstellung von Arzneimitteln zur Behandlung erektiler Dysfunktionen bei Säugetieren und Menschen.

2. Verwendung von Linsidomin gemäß Anspruch 1 in Kombination mit einem oder mehreren Wirkstoffen aus den Wirkstoffgruppen Adenosin, Vitaminen, Prostaglandinen, Calcium-Antogonisten, α-Rezeptorenblocker, Relaxantien der glatten Muskulatur zur Herstellung von Arzneimitteln zur Behandlung erektiler Dysfunktionen bei Säugetieren und Menschen.

3. Verwendung von Linsidomin und Wirkstoftkombinationen entsprechend der Ansprüche 1 bis 2, dadurch gekennzeichnet, daß 0,1 bis 5 mg Linsidomin gemäß der Ansprüche 1 bis 3 pro Dosiseinheit zur Behandlung erektiler Dysfunktionen bei Säugetieren und Menschen eingesetzt werden.

4. Einmal-Arzneimittelpackung, enthaltend in steriler Abpackung eine Einmalspritze mit einer parenteralen Zubereitung einer Dosiseinheit eines erektionsfördernden Wirkstoffes aus der Gruppe Linsidomin und seine pharmakologisch verträglichen Salze oder aus der Gruppe Linsidomin und seine pharmakologisch verträglichen Salze in Wirkstoffkombination mit synergistisch wirkenden Substanzen gemäß Patentanspruch 2, mit Desinfektionsmitteln versehene sterile Tupfer sowie Anweisungen zur Verwendung.

5. Einmal-Arzneimittelpackung gemäß Anspruch 4, dadurch gekennzeichnet, daß der Wirkstoff Linsidomin hydrochlorid ist.

6. Einmal-Arzneimittelpackung gemäß Anspruch 4 oder 5, dadurch gekennzeichnet, daß die Wirkstoffmenge 2 mg beträgt.

## Claims

1. Use of linsidomine or its pharmacologically compatible salts for a manufacture of medicaments for the treatment of erectile dysfunctions in mammals and humans.

2. Use of linsidomine according to claim 1 in combination with one or more active ingredients selected from the group of effective substances of adenosine, vitamins, prostaglandins, calcium antagonists, α-receptor blockers, relaxants of smooth muscles for a manufacture of medicaments for the treatment of erectile dysfunctions in mammals and humans.

3. Use of linsidomine and active ingredient combinations according to claims 1 to 2, characterized in that 0.1 to 5 mg of linsidomine according to claims 1 to 2 per dosage unit are used for a treatment of erectile dysfunctions in mammals and humans.

4. Single use medicament administration kit, containing, in a sterile package, a single use disposable syringe and comprising a parenteral composition of a dosage unit of an erection-promoting active ingredient selected from the group of linsidomine and its pharmacologically compatible salts or selected from the group consisting of linsidomine and its pharmacologically compatible salts in combination with synergistically effective substances according to claim 2, sterile swabs comprising a disinfectant as well as instructions for use.

5. Single use medicament administration kit according to claim 4, characterized in that the active ingredient is linsidomine hydrochloride.

6. Single use medicament administration kit according to claims 4 or 5, characterized in that the amount of active ingredient is 2 mg.

## Revendications

1. Utilisation de linsidomine ou de ses sels pharmacologiquement compatibles pour la fabrication de médicaments pour le traitement des troubles de l'érection chez le mammifère et chez l'homme.

2. Utilisation de linsidomine selon la revendication 1, en combinaison avec un ou plusieurs agents actifs parmi les groupes d'agents actifs comprenant de l'adénosine, des vitamines, des prostaglandines, des anti-calcium, des médicaments bloquant les récepteurs-α, des relaxants de la musculature lisse, servant à la fabrication de médicaments pour traiter les troubles de l'érection chez le mammifère et chez l'homme.

3. Utilisation de linsidomine et de combinaisons d'agents actifs selon les revendications 1 et 2, caractérisée en ce que l'on utilise entre 0,1 mg et 5 mg de linsidomine selon les revendications 1 à 3 pour chaque dose unitaire, pour le traitement des troubles de l'érection chez le mammifère et chez l'homme.

4. Conditionnement de médicaments à usage unique contenant, dans un emballage stérile, une seringue à usage unique, avec une préparation parentérale d'une dose unitaire d'un agent actif favorisant l'érection, l'agent actif faisant partie. du groupe de la linsidomine et de ses sels pharmacologiquement compatibles, ou faisant partie du groupe de la linsidomine et de ses sels pharmacologiquement compatibles dans une combinaison d'agents actifs avec des substances agissant par synergie selon la revendication 2, contenant des tampons stériles avec-des moyens de désinfection et comportant également des indications d'utilisation.

5. Conditionnement de médicaments à usage unique selon la revendication 4, caractérisé en ce que l'agent actif est de l'hydrochlorure de linsidomine.

6. Conditionnement de médicaments à usage unique selon la revendication 4 ou 5, caractérisé en ce que la quantité d'agent actif est de 2 mg.
